# EUROPEAN PATENT APPLICATION

(11) **EP 1 222 933 A1**
(43) Date of publication of application: **17.07.2002**
(21) Application number: 01200123.6
(22) Date of filing: 15.01.2001
(51) Int. Cl.: A61K 48/00, A61K 38/02, C07K 14/705, C07K 16/18, C12N 5/00

(54) **Generation and/or reduction of new lung tissue in an affected lung, by modulation of the Wnt-pathway**

(71) Applicant: Academisch Ziekenhuis Leiden h.o.d.n., Leids Universitair Medisch Centrum, 2333 ZA Leiden (NL)
(72) Inventor: The designation of the inventor has not yet been filed
(74) Representative: Prins, Adrianus Willem

(57) **Abstract**

The present invention provides a means to influence the formation and/or reduction of new lung cells, by influencing a Wnt-pathway in an alveolar type II cell and/or alveolar type II tumor cell from said lung. Therefore, the invention provides a composition comprising a nucleic acid capable of binding at least a functional part of a nucleic acid encoding a protein which is involved in a Wnt-pathway in said cell, said binding influencing said Wnt-pathway.

A composition of the invention may also comprise a protein capable of binding at least a functional part of a protein which is involved in a Wnt-pathway in said cell, or at least a functional part of a nucleic acid encoding a protein which is involved in a Wnt-pathway in said cell, said binding influencing said Wnt-pathway.

A composition of the invention is suitable for the preparation of a medicament against emphysema, Respiratory Distress Syndrome and/or lung cancer.

## Description

The invention relates to the field of medicine, more particularly to the treatment of lung diseases.

Worldwide, much investigation has been done on lung cells and diseases which affect lung cells, for instance emphysema and lung cancer. Until now, however, there is no efficient treatment of emphysema and lung cancer. In case of emphysema, patients suffer from shortness of breath, in first instance only on exertion, later on also at rest. This symptom may be accompanied by coughing, often with mucus expectorated. In later stages of the disease, heart failure occurs due to low oxygen levels in the blood circulation, often presenting as swollen ankles and liver enlargement. Pulmonary symptoms can be reduced by bronchodilator therapy and by use of courses of oral steroids. End-stage disease is treated with supplementation of oxygen by nasal canula. There is no treatment for the underlying cause of the disease. Consequently, most attention is being paid to decrease or even stop the process of dying of lung cells. Although some result has been obtained by the use of inhaled steroids, the lung damage continues which causes a progressive decrease in function (Pauwels et al., 1999; Burge, 2000). The problem is that even if said lung diseases can be counteracted, the lungs are already damaged by the disease. A solution to this problem would be the generation of new lung tissue. However, presently it is not possible to generate new lung tissue in a patient suffering from a lung disease.

In case of lung cancer, there are means of counteracting growth of the tumor. However, presently there is no medication which decreases the number of tumor cells in every patient. Decreasing the number of tumor cells is highly favorable, because that would actually cure the disease. Until now, there is no general effective treatment for all kinds of lung cancer.

The present invention provides a new approach to counteract diseases which affect lung cells. In one embodiment the invention provides a means to counteract diseases which decrease the number of lung cells. The present invention does not only decrease the number of dying or abnormal cells. The invention discloses the uncommon and surprising approach to influence the number of viable lung cells in an affected lung. If said number is increased, the lung is able to recover from damage caused by a disease which was not efficiently, if at all, possible before the present invention.

The invention provides a way to influence the number of lung cells by influencing a Wnt-mediated signaling pathway (referred to in this disclosure as Wnt-pathway) in said cells. The Wnt gene family encodes developmentally important secreted factors, involved in cell growth, differentiation and organogenesis (Wodartz & Nusse, 1998). Wnt signaling events are initiated by receptor activation involving binding to the cysteine-rich domain (CRD) of frizzled 7-transmembrane receptor protein (Fz) (Bhanot et al., 1996). A classical Wnt signal suppresses the activity of glycogen synthase kinase 3 (GSK-3), leading to changes in phosphorylation and increased stability of the β-catenin protein in the cytoplasm (Hinck et al., 1994). β-catenin is essential for activating target genes in response to Wnt signaling (Miller & Moon, 1996; Willert & Nusse, 1998), since it complexes with HMG box transcription factors of the TCF/LEF family (Behrens et al., 1996; Molenaar et al., 1996; Huber et al., 1996). It has been shown that the presence of proteins that are able to bind Wnt proteins through the CRD likely antagonize their actions. Amongst these are the so-called secreted Frizzled-related (sFRPs) proteins (Leyns et al., 1997; Wang et al., 1997).

Components of the Wnt signaling pathway have been found to be present during organogenesis in the mouse (Roelink & Nusse, 1991; Buhler et al., 1993; Parr et al., 1993; Christianses et al., 1995; Wang & Shackleford, 1996; Cho & Dressler, 1998; Korinek et al., 1998; Leimester et al., 1998; Oosterwegel et al., 1993). Moreover, loss of function of Wnt and Wnt-related genes leads to abnormal development in the mouse (McMahon & Bradley, 1990; Monkley et al., 1993; Takada et al., 1994; Stark et al., 1994; Galceran et al., 1999; Liu et al., 1999; Yamaguchi et al., 1999; Brisken et al., 2000; Lee et al., 2000). Several Wnts and components of the Wnt pathway are expressed in the murine lung in the course of its development (Gavin et al., 1990; Levay-young et al., 1996; Katoh et al., 1996; Lako et al., 1998; Zakin et al., 1998; Imai & D'Armiento, 1999). This shows that Wnt signaling is important for normal lung morphogenesis.

In one aspect the present invention provides a composition capable of influencing the proliferation and/or differentiation behavior of an alveolar type II cell and/or an alveolar type II tumor cell from a lung, comprising a nucleic acid capable of binding at least a functional part of a nucleic acid encoding a protein which is involved in a Wnt-pathway in said cell, said binding influencing said Wnt-pathway.

Alveolar type II cells arise at a specific stage of lung development as has been reported for the mouse (Ten Have-Opbroek, 1975; 1979; 1981; 1991) and other species including humans (Otto-Verberne and Ten Have-Opbroek, 1987; Otto-Verberne et al., 1988; Ten Have-Opbroek and Plopper, 1992). In the mouse embryo, the lung primordium appears at about 9.5 days after conception (a.c.) (Ten Have-Opbroek, 1981; 1991). It develops into the prospective trachea and two lung buds. The latter give rise to the primordial system of the right and left lungs, which is composed of primordial tubules lined by undifferentiated pseudostratified columnar epithelium. From 14.2 days a.c. onward, the primordial system differentiates into the prospective bronchial system and the prospective alveolar system (unit: pulmonary acinus).

The pulmonary acinus consists of tubules called acinar tubules (Ten Have-Opbroek, 1979). While the epithelium of the bronchial tubules is columnar, the epithelial lining of the acinar tubules is low-columnar or cuboid and composed of prospective alveolar type II cells (Ten Have-Opbroek, 1979; Ten Have-Opbroek et al., 1988). This is the so-called pseudoglandular period of lung development, which lasts until day 16.6 a.c. In later stages of lung development (i.e canalicular, terminal sac and alveolar periods), a further development of the bronchial and alveolar systems takes place, and the acinar tubules start to transform into derivative structures with a duct-, sac- or pouch-like shape. The epithelial lining of these structures now also contains flatter cells, which are prospective alveolar type I cells (Ten Have-Opbroek et al., 1990). Alveolar type II cells play an important role in the formation of the pulmonary acinus, because they are the only dividing alveolar epithelial cells and the stem cells for the alveolar type I cells. Alveolar type II cells are (one of the) predominant stem cells in the development of the two major subsets of non-small cell lung cancer, namely adenocarcinomas and squamous cell carcinomas (Ten Have-Opbroek et al., 1990; 1993; 1994; 1996; 1997; 2000).

Proliferation of an alveolar type II cell is defined as dividing of said cell, forming more cells.

Differentiation of an alveolar type II cell is defined as changing of said cell into a mature alveolar type II cell, or into another kind of cell, said other kind of cell having for instance a different shape and/or function. One example is the change of an alveolar type II cell into an alveolar type I cell.

A composition of the invention may comprise a nucleic acid capable of binding at least a functional part of a nucleic acid encoding a protein which is involved in a Wnt-pathway in said cell. Said binding influences expression of said protein. This way, said binding influences said Wnt-pathway.

Alternatively, a composition of the invention may comprise a protein which is capable of binding at least a functional part of a protein which is involved in a Wnt-pathway. Binding of a protein of the invention to said protein which is involved in a Wnt-pathway, changes the properties of said protein which is involved in a Wnt-pathway. This way, said Wnt-pathway is influenced.

A composition of the invention may also comprise a protein which is capable of binding at least a functional part of a nucleic acid encoding a protein which is involved in a Wnt-pathway in said cell. Binding of a protein of the invention to said functional part of a nucleic acid influences expression of said protein which is involved in a Wnt-pathway in said cell. Said binding, for instance, inhibits expression of said protein. This influences the Wnt-pathway.
Thus, another embodiment of the invention provides a composition capable of influencing the proliferation and/or differentiation behavior of an alveolar type II cell and/or an alveolar type II tumor cell from a lung, comprising a protein capable of binding at least a functional part of a protein which is involved in a Wnt-pathway in said cell, or at least a functional part of a nucleic acid encoding a protein which is involved in a Wnt-pathway in said cell, said binding influencing said Wnt-pathway.

A functional part of a nucleic acid is defined as a part which is essential for expression of said protein. Said functional part may for instance encode a functional part, derivative, and/or analogue of said protein.

A functional part of a protein is defined as a part which has the same kind of properties as said protein in kind, not necessarily in amount.

A functional derivative of a protein is defined as a protein which has been altered such that the properties of said derivative are essentially the same in kind, not necessarily in amount. A derivative can be provided in many ways, for instance through conservative amino acid substitution.

A person skilled in the art is well able to generate analogous compounds of a protein. This can for instance be done through screening of a peptide library. Such an analogue has essentially the same properties of said protein in kind, not necessarily in amount.

A composition of the invention may be used to generate more lung cells. This is for instance desirable if lung tissue has been damaged by a disease like emphysema. For more lung cells to be generated, a Wnt pathway may be upregulated. Thus in one aspect the invention provides a composition according to the invention, wherein said Wnt-pathway is upregulated.

In other cases, however, it may be desirable to stop proliferation and/or differentiation of lung cells. This is for instance true if an individual suffers from lung cancer. It has been found that several components of Wnt signaling are implicated in the genesis of human cancer (Morin et al., 1997, Rubinfeld et al., 1997) including lung cancer (Winn et al., 2000). Therefore, in another aspect, the present invention discloses a means of decreasing the amount of lung tumor cells by downregulating a Wnt-pathway in said tumor cells.

A composition of the invention is capable of influencing the proliferation and/or differentiation behavior of an alveolar type II cell and/or alveolar type II tumor cell. Said cells may be located inside a body of a human or animal. However, other locations (*in vitro)* are possible. So in one aspect the invention provides a composition according to the invention, wherein said cell is located inside a body of a human or animal.

A protein which is involved in a Wnt-pathway in a lung cell is for instance a secreted Frizzled-related protein. Said protein counteracts a Wnt-pathway, by binding to certain Wnt-proteins and antagonizing their actions. So, in another aspect the invention provides a composition according to the invention, which is at least in part capable of inhibiting expression of at least one secreted Frizzled-related protein. If said secreted Frizzled-related protein is less expressed, less secreted Frizzled-related protein will be present to counteract a Wnt-pathway.

Expression of a secreted Frizzled-related protein may be inhibited by a nucleic acid which is capable of binding to at least a functional part of DNA and/or RNA encoding said secreted Frizzled-related protein. Said nucleic acid may be an antisense strand. If said DNA and/or RNA encoding secreted Frizzled-related protein is bound by an antisense strand, expression of secreted Frizzled-related protein is, at least in part, inhibited. Thus in one aspect the invention provides a compound according to the invention, which at least comprises one antisense strand of at least a functional part of DNA and/or RNA encoding secreted Frizzled-related protein.

Alternatively, a Wnt-pathway may be influenced by influencing a Wnt-pathway inhibiting property of a secreted Frizzled-related protein. Expression of secreted Frizzled-related protein may remain the same in this case. In this case, the same amount of secreted Frizzled-related protein is present, but the Wnt-pathway inhibiting property of said protein has changed. Thus, in one aspect, the invention provides a compound according to the invention, which is capable of at least in part counteracting a Wnt-pathway inhibiting property of at least one secreted Frizzled-related protein.

A Wnt-pathway inhibiting property of a secreted Frizzled-related protein can be changed by binding of a compound to said secreted Frizzled-related protein. Binding of a compound to said protein can for instance alter the conformation of said protein. A person skilled in the art can think of many other ways how binding of a compound to a protein can change it's properties. Thus, another aspect of the invention discloses a compound according to the invention, which is capable of binding to at least one secreted Frizzled-related protein. Said binding compound may be an antibody. So in yet another aspect the invention provides a compound according to the invention, which comprises an antibody comprising a binding specificity against a secreted Frizzled-related protein, or a functional part, derivative and/or analogue of said antibody. A functional part, derivative and/or analogue is defined herein as disclosed above.

We have demonstrated that expression of secreted Frizzled-related protein-1 (sFRP-1), sFRP-2 and sFRP-4 in mouse embryos occurred during lung development (example 1). This suggests that at least these sFRP's are important for the proliferation and/or differentiation process of lung cells. Thus in one aspect the invention discloses a compound according to the invention, wherein said Frizzled-related protein is sFRP-1, sFRP-2, and/or sFRP-4.

We have demonstrated that transcription factors of the TCF/LEF family are also involved in lung development in a mouse (example 1). Therefore, to influence proliferation and/or differentiation of a lung cell, one embodiment of the invention provides a compound according to the invention, which is capable of activating expression of at least one transcription factor of the TCF/LEF family. Said compound may for instance be an enhancer of transcription of a gene encoding said member of the TCF/LEF family. Alternatively, said compound may be a nucleic acid encoding said member of the TCF/LEF family. If said nucleic acid is administered to a cell, expression of said member of the TCF/LEF family is increased. So one embodiment of the invention discloses a compound according to the invention, which at least comprises one nucleic acid encoding a transcription factor of the TCF/LEF family or a functional part, derivative and/or analogue thereof.

We have shown that at least transcription factors TCF-1, TCF-3, TCF-4 and/or Lef-1 are involved in lung development (table 1). Thus, one embodiment of the invention provides a compound according to the invention, wherein said transcription factor of the TCF/LEF family is TCF-1, TCF-3, TCF-4 and/or LEF-1.

Forming of new alveolar tissue in patients can be stimulated by (re)activation of formation of alveolar buds. This is an embryologic mechanism that is still active in the adult situation but at a much lower level (i.e. local concentrations of alveolar type II cells in connection with alveolar epithelial cell renewal). Formation of alveolar buds is based on active proliferation of alveolar type II cells. Formed alveolar buds proliferate into surrounding, eventually new induced, tissue. As a compound of the invention is capable of influencing said proliferation of alveolar type II cells, one embodiment of the invention provides a compound according to the invention, which is capable of inducing the formation of an alveolar bud.

Another important function of alveolar type II cells is synthesis and secretion of surfactant. Said surfactant regulates the surface tension in the alveoli. So a compound of the invention is also useful for individuals suffering from surfactant deficiency. Said individuals may suffer from Respiratory Distress Syndrome. Therefore, one embodiment of the invention provides a compound according to the invention, which is capable of inducing synthesis and/or secretion of surfactant by a lung cell.

Another embodiment of the present invention provides an isolated cell, comprising a compound according to the invention. Said compound may comprise a nucleic acid capable of binding at least a functional part of a nucleic acid encoding a protein which is involved in a Wnt-pathway in said cell. To provide a cell with said nucleic acid, said nucleic acid may be inserted into a vector. Thus, one embodiment of the invention provides a vector comprising a nucleic acid capable of binding at least a functional part of a nucleic acid encoding a protein which is involved in a Wnt-pathway in a cell, said binding influencing said Wnt-pathway.

A vector of the invention may also comprise a nucleic acid encoding a protein capable of binding at least a functional part of a protein which is involved in a Wnt-pathway in a cell, or at least a functional part of a nucleic acid encoding a protein which is involved in a Wnt-pathway in a cell, said binding influencing said Wnt-pathway.

A compound of the invention is particularly suited for the preparation of a medicament, especially for lung diseases. So in one aspect the invention provides a use of a compound according to the invention for the preparation of a medicament. Lung diseases which can be, at least in part, counteracted by a compound of the invention comprise emphysema, Respiratory Distress Syndrome, and lung cancer.

So in one aspect, the invention provides a use of a compound according to the invention for the preparation of a medicament for emphysema.

In another aspect, the invention provides a use of a compound according to the invention for the preparation of a medicament for Respiratory Distress Syndrome.

In yet another aspect, the invention provides a use of a compound according to the invention for the preparation of a medicament for lung cancer.

As a compound of the invention is capable of inducing the formation of an alveolar bud, yet another embodiment of the invention provides a method for inducing the formation of an alveolar bud, comprising administering a compound according to the invention to an alveolar type II cell.

Yet another embodiment provides a method for inducing synthesis and/or secretion of surfactant by a cell, comprising administering a compound according to the invention to said cell. Said cell may be an alveolar type II cell.

As a compound of the invention is, at least in part, capable of counteracting lung diseases like emphysema, Respiratory Distress Syndrome, and lung cancer, the invention provides in one aspect a method for, at least in part, treatment of emphysema, comprising administering a compound according to the invention to an individual.

In another aspect, the invention provides a method for, at least in part, treatment of Respiratory Distress Syndrome, comprising administering a compound according to the invention to an individual.

In yet another aspect, the invention provides a method for, at least in part, treatment of lung cancer, comprising administering a compound according to the invention to an individual.

In lung cancer, expression of components of the Wnt-pathway may be up- or down-regulated in the epithelial, mesenchymal or other cells causing enhanced proliferation of said cells. If a component of the Wnt-pathway is down-regulated (e.g. Wnt7a, Calvo et al., 2000), up-regulation of said component provides a means to slow down proliferation of said cells. This can be achieved by replacement of said component, e.g. by administration of cells manipulated to express said Wnt component (e.g. Wnt 7a). However, components of a Wnt-pathway may also be up-regulated in lung cancer cells as is the case in e.g. colon cancer cells (Bienz & Clevers, 2000). Inhibition of the activity of said components can be used to reduce proliferation of the relevant cells. This may be achieved by antisense techniques as described before, e.g. by local administration in the airways of antisense oligos for beta-catenin, or another component of the Wnt-pathway that is up-regulated.

Thus, for treatment of lung cancer, expression of a component of the Wnt-pathway may have to be either up- or down-regulated, depending on the particular component.

The following, non-limiting, examples are meant to illustrate the invention. A person skilled in the art is capable to perform alternative experiments which are still in the scope of the present invention.

### EXAMPLES

### Example 1. Expression of TCF/LEF transcription factors, β-catenin and secreted Frizzled-related proteins in alveolar epithelium and/or surrounding mesenchyme during murine lung development.

### Animals

In this study, an inbred Swiss-type mouse strain with a gestation time of about 19 days after conception (a.c.) was used. The embryos were obtained from female mice aged about 3 months and weighing 30-40 g. They carried 8 to 12 embryos, whose weight was used as a parameter of the developmental stage since it is a more sensitive indicator than the age in days a.c. A growth curve based on the relationship between weights and ages allowed us to determine what we call the "developmental age" of the mouse embryo (Goedbloed, 1976; Ten Have-Opbroek et al., 1988), which is indicated in the text for all embryos used.

### Processing of the tissue

The pregnant mice were anesthetized with an intraperitoneal injection of 2% anexin (Rompun):10% ketamin (Nimatek) (1:1) and killed by cervical dislocation. The embryos were removed from the uterus and weighed to determine the developmental age. Then the lungs were removed from the mother and the embryos by thoracotomy, divided in two portions (the left lung consisting of one large lobe, and the right lung composed of four lobes) and fixed by immersion in 4% paraformaldehyde overnight (o/n) at room temperature (rt) with rocking.

### Whole mount in situ hybridisation (ISH) probes

Both antisense and sense digoxigenin-labeled RNA probes were generated from LEF-1, TCF-1, TCF-3 and TCF-4 cDNAs, and from sFRP-1, sFRP-2, sFRP-3 and sFRP-4 cDNAs.

### Whole mount ISH

After washing for 5 min in PBT (PBS containing 0.1% Tween-20), the specimens were dehydrated through a graded methanol series (25%, 50% and 75% in PBT for 5 min each, and 100% 2x for 5 min) and stored in methanol 100% at - 20°C until use.

Whole mount ISH was performed essentially as described (Wilkinson and Nieto 1993, Wilkinson, 1995; Nieto et al., 1996) with minor modifications. Afterwards, the whole mount ISH samples were sectioned and mounted on slides to study the cellular localization of the mRNA signal.

### Immunohistochemistry

Immunohistochemical staining was performed using the avidin-biotin complex (ABC) method with peroxidase labeling and 3-3'diaminobenzidine (DAB) as the chromogen (VECTOR; Burlingame, CA, USA). Briefly, the procedure involves the following steps: 1) hydration of the paraffin sections through xylene and a graded ethanol series (100-70%, each step lasting 30 min) and quenching of the endogenous peroxidase activity with 100% methanol containing 0.4% hydrogen peroxide (H₂O₂) for 20 min at rt; 2) 3 times rinsing in Tris Maleate buffer (TMB, pH 7.6) for 1 min at rt and incubation with 10% normal horse serum for 1 h at rt; 3) incubation with the primary antibody (anti β-catenin, anti LEF-1/TCFs and anti sFRPs; all diluted in PBS, pH 7.6) overnight at 4°C and rinsing in TMB; 4) incubation with a 1:400 dilution of biotinylated swine anti-rabbit IgG (DAKO, Denmark) or biotinylated horse anti-mouse IgG for 60 min at rt and rinsing in TMB; 5) incubation with ABC for 30 min at rt and rinsing in TMB; and 6) incubation with TMB containing 0.04% DAB and 0.006% H₂O₂ for 10 min at rt. Finally, the sections were washed in TMB for 1 min and in tap water for 10 min, then counterstained with hematoxylin for 5 sec, rinsed in tap water for 10 min, dehydrated through a graded ethanol series (70-100%) and xylene and mounted with xylene-soluble mounting medium *Depex* (H.D. SUPPLIES, England).

Immunohistochemical controls were performed on the serial mouse fetal lung sections using normal rabbit or mouse serum as the primary antibody, or omission of one of the incubation steps.

### Example 2. Activation of alveolar type II cells in a murine lung by influencing a Wnt-pathway

To provide proof of evidence, a fetal lung organoid culture (obtained from a mouse) is used. Generation of new alveolar tissue in patients (see p. 9) can be stimulated by activation or re-activation of alveolar bud formation. Alveolar bud formation is a general growth principle in both the fetal and the adult mammalian lung. As a proof of evidence, it is therefore shown that manipulation of expression and/or function of selected molecules, involved in a Wnt-pathway, stimulates the process of alveolar bud formation. Activation of alveolar type II cells by influencing a Wnt-pathway is for instance demonstrated using anti-sense oligonucleotides. These oligonucleotides may be directed against sFRPs. One subject of investigation is the means of administration of compositions capable of influencing a Wnt-pathway. The effect of administration is investigated using a biological *in vitro* test-system, preferably the above-mentioned fetal murine organoid lung culture. Complementary testing can be performed in the adult mouse lung using for instance an organoid culture.

The fetal murine lung culture is generated using the protocol of prof. Zimmermann, Freie Universität, Berlin (Zimmermann, 1987; Zimmermann, 1989; Hundertmark et al., 1999). The presence of molecules involved in a Wnt-pathway in said fetal murine lung culture is tested using molecular-biological methods, as for instance *in situ* hybridisation and/or immunohistochemistry. Once said molecules involved in a Wnt-pathway are found, anti-sense oligonucleotides against said molecules are generated. Modified stable anti-sense oligonucleotides are produced using existing protocols (Augustine et al., 1995; Dagle et al., 2000; Heasman et al., 2000). After that, the *in vitro* effect of said generated oligonucleotides is tested in the fetal or adult murine lung culture. Said oligonucleotides are administered to the culture medium in different concentrations. The effective concentration of the administered oligonucleotides, capable of influencing formation of alveolar tissue, is determined experimentally using morphological and/or biochemical techniques. For instance, sections (5 mm) from treated alveolar tissues and untreated controls are investigated by immunohistochemistry and/or morphometry. Criteria are for instance the ratio between primordial lung cells and alveolar type II cells in the lung buds, and/or the increase of the number of alveolar type II cells, and/or proliferating alveolar type II cells, per cm basal membrane. Other criteria include the number of alveolar spaces, the size of the gas exchange surface, and the weight and/or volume of the lung (Otto-Verberne et al., 1991; Brandsma et al., 1994; Heemskerk-Gerritsen et al., 1996). Additional information on alveolar type II cell differentiation is obtained by electronmicroscopic research and/or by biochemical investigation, like for instance surfactant protein A (SP-A) detection in the culture medium.

Preferably, results concerning the formation of alveolar tissue are obtained using oligonucleotides, or combinations of oligonucleotides, which disturb the type II cell equilibrium. More preferably, said oligonucleotides inhibit differentiation in favour of proliferation.

### Results:

### Example 1. Expression of TCF-1, TCF-3, TCF-4 and LEF-1 mRNA during murine lung development (Table 1).

### 1) Whole mount ISH data:

TCF-1 mRNA was clearly expressed around 11 days a.c., and it reached the maximum levels between 13 and 15 days a.c. Interestingly, TCF-1 mRNA expression remained slightly positive through 16, 17 and 18 days a.c., and also in the adult lung. mRNA coding for TCF-3 was found to be expressed as early as 10 days a.c. Its expression levels were high from 12 days a.c. till 16 days a.c., and began to decrease between 17 and 18 days a.c. Regarding TCF-4 mRNA expression, similar to those of TCF-3, it was present already at 10 days a.c. and achieved the highest levels around 12 days a.c. However, in contrast to the other transcription factors studied, at 13 days a.c. the TCF-4 mRNA expression declined and it was nearly negative at 14 days a.c. Finally, mRNA coding for LEF-1 was found positive at 11 days a.c. The signal was elevated during 12, 13, 14 and 15 days a.c. At 16 days a.c., LEF-1 mRNA expression decreased and was negative at 17 d.a.c.

### 2) Sections of the whole mount ISH samples:

The sectioning of the whole mount ISH samples showed the cellular localization of the mRNA expression for the TCFs/LEF-1 transcription factors. TCF-1 mRNA expression appeared to be located in the mesenchymal cells in close proximity to the alveolar epithelial cells, but also in the apical cytoplasmic areas of the epithelial cells lining the lung primordia and acinar tubules. For TCF-3, the mRNA expression was present mainly in the apical side of the alveolar epithelial cells, similar to the signal corresponding to TCF-4 mRNA. Finally, LEF-1 mRNA expression was located just in the mesenchyme around the epithelial lining of the lung primordia and acinar tubules.

### Protein expression of β-catenin, LEF1/TCFs and sFRPs during murine lung development.

At 13 days a.c., the protein expression corresponding to β-catenin was found to be present in the cell junctions of the prospective bronchial epithelium, while the alveolar epithelial cells lining the acinar tubules (prospective respiratory epithelium) showed β-catenin protein expression in the cytoplasm as well as in the nuclei. Later on during development (around 17 days a.c.), the differentiating alveolar type I cells were negative for the expression of this protein, while some alveolar type II cells were still positive.

LEF-1/TCFs protein cytoplasmic expression was present in the epithelial cells (prospective bronchial and respiratory epithelium and/or in the mesenchyme) at 13 days a.c. At 17 days a.c., some TCF expression was still present in the alveolar type II cells.

For sFRP-protein, a slight expression was located mainly in the cytoplasm of the epithelial cells lining both the prospective bronchial and respiratory epithelium, but also in the mesenchyme, at 13 days a.c. The alveolar type II cells together with the differentiating alveolar type I cells were found to be negative for sFRPs protein expression at 17 days a.c.

### Expression of sFRP-1, sFRP-2, sFRP-3 and sFRP-4 mRNA during murine lung development (Table 2).

### Whole mount ISH data:

Both sFRP-1 and sFRP-2 were found to be expressed early in the embryonic lung, while sFRP-3 was not present at any developmental age. SFRP-1 and sFRP-2 mRNA expression was present at 10 days a.c., persisted through 11 and 12 days a.c., and declined around 13 days a.c. As deduced from the whole mount expression pattern, it was located in the connective tissue around the epithelial cells of the lung buds and primordia. For sFRP-4, the mRNA was found during the same period of embryonic development, but the expression pattern indicated an epithelial localization, notably in the apical side of the cytoplasm.

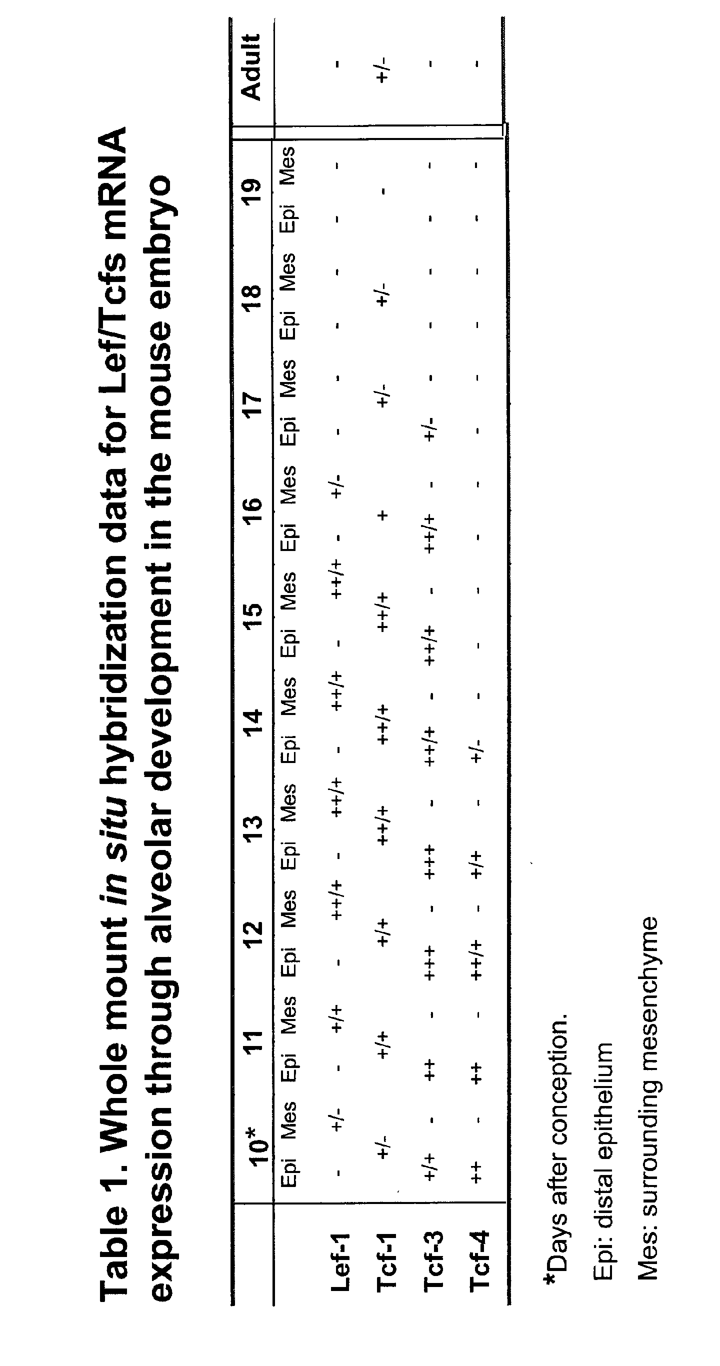

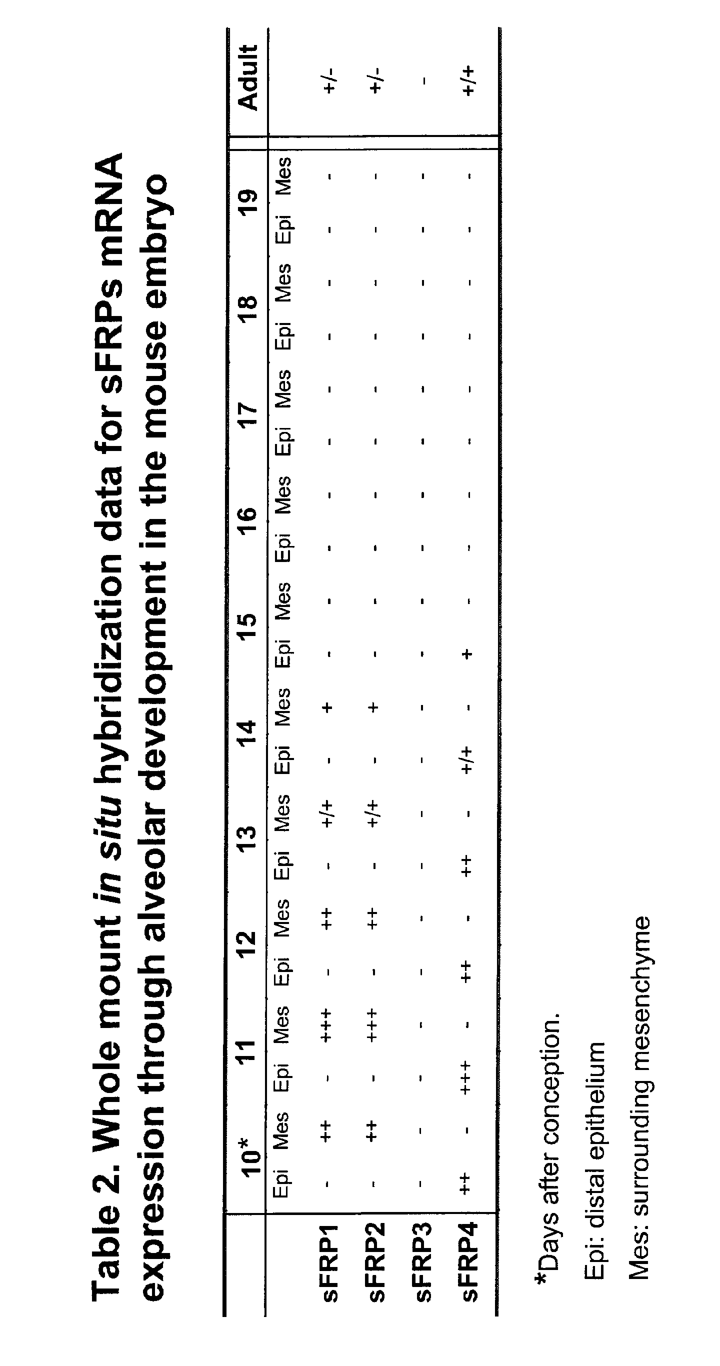

### References

Augustine K, Liu ET and Sadler TW. 1993. Antisense attenuation of Wnt-1 and Wnt-3a expression in whole embryo culture reveals roles for these genes in craniofacial, spinal cord, and cardiac morphogenesis. Dev Genet, 14:500-20.

Augustine KA, Liu ET, Sadler TW. 1995. Interactions of Wnt-1 and Wnt-3a are essential for neural tube patterning. Teratology 2: 107-19.

Barker N and Clevers H. 2000. Catenins, Wnt signaling and cancer. Bioessays, 22:961-965.

Behrens J, von Kries JP, Kuhl M, Bruhn L, Wedlich D, Grosschedl R and Birchmeier W. 1996. Functional interaction of beta-catenin with the transcription factor LEF-1. Nature, 382:638-42.

Bhanot P, Brink M, Samos CH, Hsieh JC, Wang Y, Macke JP, Andrew D, Nathans J and Nusse R. 1996. A new member of the frizzled family from Drosophila functions as a Wingless receptor. Nature, 382:225-30.

Bienz M, Clevers H. 2000 Linking colorectal cancer to Wnt signaling. Cell.103:311-20.

Brandsma AE, Ten Have-Opbroek AAW, Vulto IM, Molenaar JC, Tibboel, D. 1994. Alveolar epithelial composition and architecture of the late fetal pulmonary acinus. An immunocytochemical and morphometric study in a rat model of pulmonary hypoplasia and congenital diaphragmatic hernia. Exp Lung Res 20:491-515.

Brisken C, Heineman A, Chavarria T, Elenbaas B, Tan J, Dey SK, McMahon JA,McMahon AP, Weinberg RA. 2000 Essential function of Wnt-4 in mammary gland development downstream of progesterone signaling.Genes Dev. 14:650-4.

Brown JD, Hallagan SE, McGrew LL, Miller JR and Moo RT. 2000. The maternal Xenopus beta-catenin signaling pathway, activated by frizzled homologs, induces goosecoid in a cell non-autonomous manner. Dev Growth Differ, 42:347-57.

Buhler TA, Dale TC, Kieback C, Humphreys RC, Rosen JM. 1993 Localization and quantification of Wnt-2 gene expression in mouse mammary development.
Dev Biol.155:87-96.

Burge PS, Brit Med J 2000; 320:1297-1303

Calvo R, West J, Franklin W, Erickson P, Bemis L, Li E, Helfrich B, Bunn P,Roche J, Brambilla E, Rosell R, Gemmill RM, Drabkin HA. 2000 Altered HOX and WNT7A expression in human lung cancer. Proc Natl Acad Sci U S A. 97:12776-81

Cho EA, Dressler GR. 1998 TCF-4 binds beta-catenin and is expressed in distinct regions of the embryonic brain and limbs. Mech Dev. 77:9-18

Christiansen JH, Dennis CL, Wicking CA, Monkley SJ, Wilkinson DG and Wainwright BJ. 1995. Murine Wnt-11 and Wnt-12 have temporally and spatially restricted expression patterns during embryonic development. Mech Dev, 51:341-50.

Dagle JM, Littig JL, Sutherland LB, Weeks DL. 2000. Targeted elimination of zygotic messages in Xenopus laevis embryos by modified oligonucleotides possessing terminal cationic linkages. Nucl Acids Res 28: 2153-2157.

Galceran J, Farinas I, Depew MJ, Clevers H, Grosschedl R.1999 Wnt3a-/--like phenotype and limb deficiency in Lef1(-/-)Tcf1(-/-) mice. Genes Dev. 13:709-17

Gavin BJ, McMahon JA, McMahon AP. 1990 Expression of multiple novel Wnt-1/int-1-related genes during fetal and adult mouse development. Genes Dev. 12B:2319-32.

Goedbloed JF. 1976. Embryonic and postnatal growth of rat and mouse. IV. Prenatal growth of organs and tissues: age determination, and general growth pattern. Acta Anat (Basel), 95:8-33.

Ten Have-Opbroek AAW. Immunological study of lung development in the mouse embryo. I. Appearance of a lung-specific antigen, localized in the great alveolar cell. Dev Biol 46:390-403, 1975.

Ten Have-Opbroek AAW. Immunological study of lung development in the mouse embryo. II. First appearance of the great alveolar cell, as shown by immunofluorescence microscopy. Dev Biol 69:408-423, 1979.

Ten Have-Opbroek AAW. The development of the lung in mammals: An analysis of concept and findings. Am J Anat 162:201-219, 1981.

Ten Have-Opbroek AAW, Dubbeldam JA, Otto-Verberne CJM. Ultrastructural features of type II alveolar epithelial cells in early embryonic mouse lung. Anat Rec 221:846-853, 1988.

Ten Have-Opbroek AAW, Otto-Verberne CJM, Dubbeldam JA. Ultrastructural characteristics of inclusion bodies of type II cells in late embryonic mouse lung. Anat Embryol 181:317-323, 1990.

Ten Have-Opbroek AAW, Hammond WG, Benfield JR. Bronchiolo-alveolar regions in adenocarcinoma arising from canine segmental bronchus. Cancer Letters 55:177-182, 1990.

Ten Have-Opbroek AAW. *Invited review.* Lung development in the mouse embryo. Exp Lung Res 17:111-130, 1991.

Ten Have-Opbroek AAW, Plopper CG. Morphogenetic and functional activity of type II cells in early fetal Rhesus monkey lungs. A comparison between primates and rodents. Anat Rec 234:93-104, 1992.

Ten Have-Opbroek AAW, Hammond WG, Benfield JR, Teplitz RL, Dijkman JH. Expression of alveolar type II cell markers in acinar adenocarcinomas and adenoid-cystic carcinomas arising from segmental bronchi. A study in a heterotopic bronchogenic carcinoma model in dogs. Am J Pathol 142:1251-1264, 1993.

Ten Have-Opbroek AAW, Benfield JR, Hammond WG, Teplitz RL, Dijkman JH. *Invited review.* In favour of an oncofoetal concept of bronchogenic carcinoma development. Histol Histopath 9:375-384, 1994.

Ten Have-Opbroek AAW, Benfield JR, Hammond WG, Dijkman JH. Alveolar stem cells in canine bronchial carcinogenesis. Cancer Lett 101:211-217, 1996.

Ten Have-Opbroek AAW, Benfield JR, Van Krieken JHJH, Dijkman JH. The alveolar type II cell is a pluripotential stem cell in the genesis of human adenocarcinomas and squamous cell carcinomas. Histol Histopathol 12:319-336, 1997.

Ten Have-Opbroek AAW, Shi X-B, Gumerlock PH. 3-Methylcholanthrene triggers the differentiation of alveolar tumor cells from canine bronchial basal cells and an altered p53 gene promotes their clonal expansion. Carcinogenesis 21:1477-1484, 2000.

Heasman J, Kofron M, Wylie C. 2000. Beta-catenin signaling activity dissected in the early Xenopus embryo: a novel antisense approach. Dev Biol 222:124-134.

Heemskerk-Gerritsen BAM, Dijkman JH, Ten Have-Opbroek AAW. 1996. Stereological methods: A new approach in the assessment of pulmonary emphysema. Microsc Res Techn 34:556-562.

Hinck L, Nelson WJ and Papkoff J. 1994. Wnt-1 modulates cell-cell adhesion in mammalian cells by stabilizing beta-catenin binding to the cell adhesion protein cadherin. J Cell Biol, 124:729-41.

Huber O, Korn R, McLaughlin J, Ohsugi M, Herrmann BG, Kemler R.1996 Nuclear localization of beta-catenin by interaction with transcription factor LEF-1.Mech Dev.59:3-10

Huelsken J, Vogel R, Brinkmann V, Erdmann B, Birchmeier C and Birchmeier W. 2000. Requirement for beta-catenin in anterior-posterior axis formation in mice. J Cell Biol, 148:567-78.

Hundertmark S et al. 1999. Effect of dexamethasone, triiodothyronine and dimethyl-isopropyl thyronine on lung maturation of the fetal rat lung. J Perinat Med 27:309-315.

Imai K, D'Armiento J. Expression of Wnt10b and sFRP1 in embryonic mouse lung. Am Rev Resp Crit Care Med 159:A817, 1999

Katoh M, Hirai M, Sugimura T, Terada M. 1996 Cloning, expression and chromosomal localization of Wnt-13, a novel member of the Wnt gene family. Oncogene. 13:873-6

Kispert A, Vainio S and McMahon AP. 1998. Wnt-4 is a mesenchymal signal for epithelial transformation of metanephric mesenchyme in the developing kidney. Development, 125:4225-34.

Korinek V, Barker N, Willert K, Molenaar M, Roose J, Wagenaar G, Markman M, Lamers W, Destree O and Clevers H. 1998. Two members of the Tcf family implicated in Wnt/beta-catenin signaling during embryogenesis in the mouse. Mol Cell Biol, 18:1248-56.

Lako M, Strachan T, Bullen P, Wilson DI, Robson SC and Lindsay S.1998. Isolation, characterisation and embryonic expression of WNT11, a gene which maps to 11q13.5 and has possible roles in the development of skeleton, kidney and lung. Gene, 219:101-10.

Lee SM, Tole S, Grove E and McMahon AP. 2000. A local Wnt-3a signal is required for development of the mammalian hippocampus. Development, 127:457-67.

Leimeister C, Bach A, Gessler M. 1998 Developmental expression patterns of mouse sFRP genes encoding members of the secreted frizzled related protein family. Mech Dev. 75):29-42

Levay-Young BK and Navre M. 1992. Growth and developmental regulation of wnt-2 (irp) gene in mesenchymal cells of fetal lung. Am J Physiol, 262(6 Pt 1):L672-83.

Leyns L, Bouwmeester T, Kim SH, Piccolo S and De Robertis EM. 1997. Frzb-1 is a secreted antagonist of Wnt signaling expressed in the Spemann organizer. Cell, 88:747-56.

Liu P, Wakamiya M, Shea MJ, Albrecht U, Behringer RR, Bradley A. 1999 Requirement for Wnt3 in vertebrate axis formation Nat Genet. 22:361-5

McMahon AP, Bradley A. 1990 The Wnt-1 (int-1) proto-oncogene is required for development of a large region of the mouse brain.Cell. 62:1073-85

McMahon AP, Gavin BJ, Parr B, Bradley A and McMahon JA.1992. The Wnt family of cell signalling molecules in postimplantation development of the mouse. Ciba Found Symp, 165:199-212; discussion 212-8.

Miller JR, Moon RT. 1996 Signal transduction through beta-catenin and specification of cell fate during embryogehesis.Genes Dev. 10:2527-39

Molenaar M, van de Wetering M, Oosterwegel M, Peterson-Maduro J, Godsave S, Korinek V, Roose J, Destree O, Clevers H.1996 XTcf-3 transcription factor mediates betacatenin-induced axis formation in Xenopus embryos. Cell. 86:391-9

Monkley SJ, Delaney SJ, Pennisi DJ, Christiansen JH, Wainwright BJ.1996 Targeted disruption of the Wnt2 gene results in placentation defects. Development. 122:3343-53

Morin PJ, Sparks AB, Korinek V, Barker N, Clevers H, Vogelstein B, Kinzler KW. 1997 Activation of betacatenin-Tcf signaling in colon cancer by mutations in beta-catenin or APC. Science. 275:1787-90.

Nieto MA, Patel K and Wilkinson DG. 1996. In situ hybridization analysis of chick embryos in whole mount and tissue sections. Methods Cell Biol, 51:219-35.

Nusse R and Varmus HE. 1992. Wnt genes. Cell, 69:1073-87.

Oosterwegel M, van de Wetering M, Timmerman J, Kruisbeek A, Destree O, Meijlink F, Clevers H. Differential expression of the HMG box factors TCF-1 and LEF-1 during murine embryogenesis.Development. 118:439-48

Otto-Verberne CJM, Ten Have-Opbroek AAW. Development of the pulmonary acinus in fetal rat lung: a study based on an antiserum recognizing surfactant-associated proteins. Anat Embryol 175:365-373, 1987.

Otto-Verberne CJM, Ten Have-Opbroek AAW, Balkema JJ, Franken C. Detection of the type II cell or its precursor before week 20 of human gestation, using antibodies against surfactant-associated proteins. Anat Embryol 178:29-39, 1988.

Otto-Verberne CJM, Ten Have-Opbroek AAW, De Vries ECP. Expression of the major surfactant-associated protein, SP-A, in type II cells of human lung before 20 weeks of gestation. Eur J Cell Biol 53:13-19, 1990.

Otto-Verberne CJM, Ten Have-Opbroek AAW, Willems LNA, Franken C, Kramps JA, Dijkman JH. 1991. Lack of type II cells and emphysema in human lungs. Eur Respir J 4:316-323.

Parr BA, Shea MJ, Vassileva G, McMahon AP. Mouse Wnt genes exhibit discrete domains of expression in the early embryonic CNS and limb buds.Development. 119:247-61

Pauwels RA et al., New Engl J Med 1999: 340:1948-1953

Polakis P. 2000. Wnt signaling and cancer. Genes Dev, 14:1837-51.

Porter JD and Baker RS.1997. Absence of oculomotor and trochlear motoneurons leads to altered extraocular muscle development in the Wnt-1 null mutant mouse. Brain Res Dev Brain Res, 100:121-6.

Roelink H, Nusse R.1991 Expression of two members of the Wnt family during mouse development restricted temporal and spatial patterns in the developing neural tube. Genes Dev. 3:381-8

Rubinfeld B, Albert I, Porfiri E, Munemitsu S and Polakis P. 1997. Loss of beta-catenin regulation by the APC tumor suppressor protein correlates with loss of structure due to common somatic mutations of the gene. Cancer Res, 57:4624-30.

Sarkar L and Sharpe PT. 1999. Expression of Wnt signalling pathway genes during tooth development. Mech Dev, 85:197-200.

Stark K, Vainio S, Vassileva G and McMahon AP.1994. Epithelial transformation of metanephric mesenchyme in the developing kidney regulated by Wnt-4. Nature, 372:679-83.

Takada S, Stark KL, Shea MJ, Vassileva G, McMahon JA and McMahon AP. 1994. Wnt-3a regulates somite and tailbud formation in the mouse embryo. Genes Dev, 8:174-89.

Wang J, Shackleford GM.Murine Wnt10a and Wnt10b 1996 cloning and expression in developing limbs, face and skin of embryos and in adults.Oncogene. 13:1537-44

Wang S, Krinks M, Lin K, Luyten FP and Moos M Jr. 1997. Frzb, a secreted protein expressed in the Spemann organizer, binds and inhibits Wnt-8. Cell, 88:757-66.

Wilkinson DG. 1995. RNA detection using non-radioactive in situ hybridization. Curr Opin Biotechnol, 6:20-23.

Wilkinson DG and Nieto MA. 1993. Detection of messenger RNA by in situ hybridization to tissue sections and whole mounts. Methods Emzymol, 225:361-373.

Willert K and Nusse R. 1998. Beta-catenin: a key mediator of Wnt signaling. Curr Opin Genet Dev, 8:95-102.

Winn R.A. and West J.B. 2000. Evidence for involvement of the Wnt-pathway in lung cancer.
Am.J.Respir.Crit.Care.Med. 161, A670.

Wodarz A and Nusse R. 1998. Mechanisms of Wnt signaling in development. Annu Rev Cell Dev Biol, 14:59-88.

Yamaguchi TP, Bradley A, McMahon AP, Jones S. 1999 A Wnt5a pathway underlies outgrowth of multiple structures in the vertebrate embryo.Development 126:1211-23.

Zakin LD, Mazan S, Maury M, Martin N, Guenet JL, Brulet P. 1998 Structure and expression of Wnt13, a novel mouse Wnt2 related gene. Mech Dev. 73:107-16

Zimmermann B. 1987. Lung organoid culture. Differentiation 36: 86-109.

Zimmermann B. 1989. Secretion of lamellar bodies in type II pneumocytes in organoid culture: Effects of colchicine and cytochalasin B. Exp Lung Res 15:31-47.

## Claims

1. A composition capable of influencing the proliferation and/or differentiation behavior of an alveolar type II cell and/or an alveolar type II tumor cell from a lung, comprising a nucleic acid capable of binding at least a functional part of a nucleic acid encoding a protein which is involved in a Wnt-pathway in said cell, said binding influencing said Wnt-pathway.

2. A composition capable of influencing the proliferation and/or differentiation behavior of an alveolar type II cell and/or an alveolar type II tumor cell from a lung, comprising a protein capable of binding at least a functional part of a protein which is involved in a Wnt-pathway in said cell, or at least a functional part of a nucleic acid encoding a protein which is involved in a Wnt-pathway in said cell, said binding influencing said Wnt-pathway.

3. A composition according to claim 1 or 2, wherein said Wnt-pathway is upregulated.

4. A composition according to anyone of claims 1-3, wherein said cell is located inside a body of a human or animal.

5. A composition according to anyone of claims 1-4, which is at least in part capable of inhibiting expression of at least one secreted Frizzled-related protein.

6. A compound according to claim 5, which at least comprises one antisense strand of at least a functional part of DNA and/or RNA encoding secreted Frizzled-related protein.

7. A compound according to anyone of claims 1-6, which is capable of at least in part counteracting a Wnt-pathway inhibiting property of at least one secreted Frizzled-related protein.

8. A compound according to anyone of claims 1-7, which is capable of binding to at least one secreted Frizzled-related protein.

9. A compound according to anyone of claims 1-8, which comprises an antibody comprising a binding specificity against a secreted Frizzled-related protein, or a functional part, derivative and/or analogue of said antibody.

10. A compound according to anyone of claims 5-9, wherein said Frizzled-related protein is sFRP-1, sFRP-2, and/or sFRP-4.

11. A compound according to anyone of claims 1-10, which is capable of activating expression of at least one transcription factor of the TCF/LEF family.

12. A compound according to anyone of claims 1-11, which at least comprises one nucleic acid encoding a transcription factor of the TCF/LEF family or a functional part, derivative and/or analogue thereof.

13. A compound according to claim 11 or 12, wherein said transcription factor of the TCF/LEF family is TCF-1, TCF-3, TCF-4 and/or LEF-1.

14. A compound according to anyone of claims 1-13, which is capable of inducing the formation of an alveolar bud.

15. A compound according to anyone of claims 1-14, which is capable of inducing synthesis and/or secretion of surfactant by a lung cell.

16. An isolated cell, comprising a compound according to anyone of claims 1-15.

17. A vector comprising a nucleic acid capable of binding at least a functional part of a nucleic acid encoding a protein which is involved in a Wnt-pathway in a cell, said binding influencing said Wnt-pathway.

18. A vector comprising a nucleic acid encoding a protein capable of binding at least a functional part of a protein which is involved in a Wnt-pathway in a cell, or at least a functional part of a nucleic acid encoding a protein which is involved in a Wnt-pathway in a cell, said binding influencing said Wnt-pathway.

19. Use of a compound according to anyone of claims 1-15 for the preparation of a medicament.

20. Use of a compound according to anyone of claims 1-15 for the preparation of a medicament for emphysema.

21. Use of a compound according to anyone of claims 1-15 for the preparation of a medicament for Respiratory Distress Syndrome.

22. Use of a compound according to anyone of claims 1-15 for the preparation of a medicament for lung cancer.

23. A method for inducing the formation of an alveolar bud, comprising administering a compound according to anyone of claims 1-15 to an alveolar type II cell.

24. A method for inducing synthesis and/or secretion of surfactant by a cell, comprising administering a compound according to anyone of claims 1-15 to said cell.

25. A method according to claim 24, wherein said cell is an alveolar type II cell.

26. A method for, at least in part, treatment of emphysema, comprising administering a compound according to anyone of claims 1-15 to an individual.

27. A method for, at least in part, treatment of Respiratory Distress Syndrome, comprising administering a compound according to anyone of claims 1-15 to an individual.

28. A method for, at least in part, treatment of lung cancer, comprising administering a compound according to anyone of claims 1-15 to an individual.
